# EUROPÄISCHE PATENTSCHRIFT

(11) **EP 1 650 180 B1**
(45) Veröffentlichungstag und Bekanntmachung des Hinweises auf die Patenterteilung: **21.05.2008**
(21) Anmeldenummer: 05021977.3
(22) Anmeldetag: 08.10.2005
(51) Int. Cl.: C07C 29/36, C07C 35/04

(54) **Verfahren zur Herstellung von Cyclopropanderivaten mit speziellen Metallalkoxiden**
Process for the preparation of cyclopropane derivatives with special metal oxides
Procédé de préparation de dérivés de cyclopropane avec des oxides métalliques spéciaux

(30) Priorität: 20.10.2004 DE 102004051028
(43) Veröffentlichungstag der Anmeldung: 26.04.2006
(73) Patentinhaber: H.C. Starck GmbH, 38642 Goslar (DE)
(72) Erfinder: Reuter, Knud, Dr., 47800 Krefeld (DE)
(74) Vertreter: Clauswitz, Kai-Uwe Wolfram

(56) Entgegenhaltungen:
- DATABASE WPI Section Ch, Week 199037 Derwent Publications Ltd., London, GB; Class E15, AN 1990-280970 XP002359791 & SU 1 525 140 A (BELORUSSIAN LENIN UNIV) 30. November 1989 (1989-11-30)
- MEIJERE A D ET AL: "Titanium-mediated syntheses of cyclopropylamines" JOURNAL OF ORGANOMETALLIC CHEMISTRY, ELSEVIER-SEQUOIA S.A. LAUSANNE, CH, Bd. 689, Nr. 12, 15. Juni 2004 (2004-06-15), Seiten 2033-2055, XP004509028 ISSN: 0022-328X

## Beschreibung

Die vorliegende Erfindung betrifft ein neues Verfahren zur Herstellung von CyclopropanDerivaten, und zwar Aminocyclopropanen und insbesondere Cyclopropanolen.

Zur Herstellung von Cyclopropanderivaten sind zahlreiche Synthesen bekannt. Zur Umwandlung von Alkancarbonsäurestern in Cyclopropanole wurde in jüngerer Zeit insbesondere die nach ihrem Entdecker als Kulinkovich-Reaktion bezeichnete Umsetzung von Alkancarbonsäureestern mit Grignard-Verbindungen in Gegenwart ggf. katalytischer Mengen an Titanisopropylat(en) bekannt. In der Variante dieser Reaktion nach de Meijere werden Carbonsäureamide mit Grignard-Verbindungen in Gegenwart von Titanisopröpylat(en) zu Aminocyclopropanen umgesetzt. Aktuelle Übersicht über beide Reaktionstypen findet sich in Nachrichten aus der Chemie, Bd. 52, S. 805 bis 808 (2004) und in Journal of Organometallic Chemistry, Bd. 689, S.2033-2055(2004).

Cyclopropan-Derivate spielen eine technisch außerordentlich bedeutsame Rolle, beispielsweise auf dem Gebiet der Wirkstoffe im pharmazeutischen Bereich oder für den Pflanzenschutz (z.B. Pyrethroid-Insektizide). Es besteht nach wie vor ein Bedarf an neuen Cyclopropan-Synthesen, mit denen die Möglichkeiten auf diesem Gebiet erweitert werden können.

Überraschend wurde nun gefunden, dass mit Alkoxiden von Metallen der fünften Nebengruppe, und zwar Niob und insbesondere Tantal, wirksame Reagenzien zur Verfügung stehen, die eine Cyclopropanierung von Carbonsäureestern durch Grignard-Verbindungen leisten. Damit wird das Spektrum der Kulinkovich-Reaktion erheblich erweitert, wobei die damit verbundene Verallgemeinerung des Reaktionsprinzips über die Titanverbindungen hinaus zahlreiche neue präparative Möglichkeiten erschließt.

Gegenstand der Erfindung ist somit ein Verfahren zur Herstellung von Cyclopropanderivaten der Formel I, 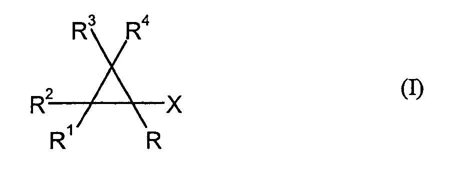 in der
- R, R¹, R², R³ und R⁴: unabhängig voneinander Wasserstoff, C₁- bis C₁₈-Alkyl (einschließlich Cycloalkyl) oder C₆- bis C₁₀-Aryl bedeuten, wobei jeweils ein Rest aus R¹, R² und ein Rest aus R³, R⁴ gemeinsam einen Cycloalkan-Ring mit 4 bis 18 C-Atomen bilden können, X entweder OH oder YR⁵R⁶ bedeutet, wobei Y entweder O oder N bedeutet und für den Fall, dass Y = O ist, R⁶ entfällt und X zwangsläufig gleich OH ist, und wobei R⁵ und R⁶ unabhängig voneinander C₁- bis C₁₈-Alkyl oder C₆- bis C₁₀-Aryl bedeuten,
das dadurch gekennzeichnet ist, dass Alkancarbonsäurederivate der Formel (II) 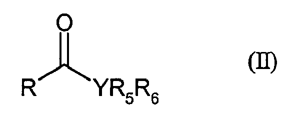 in der
- Y, R, R⁵ und R⁶: die oben zu Formel (I) angegebene Bedeutung haben.
in Gegenwart von Verbindungen von Metallen der V. Nebengruppe (M = V, Nb, Ta) der Formel (III)

HalₙM(OR⁷)₅₋ₙ (III)

in der
- Hal: ein Halogen aus der Gruppe Chlor, Brom oder Iod, bevorzugt Chlor ist, n eine ganze Zahl von 0 bis 5, bevorzugt 0 oder 1, besonders bevorzugt 0 ist, M ein Element aus der Gruppe Vanadium, Niob und Tantal, bevorzugt Niob und Tantal, besonders bevorzugt Tantal ist und R⁷ ein C₁- bis C₈- Alkylrest ist
mit Grignardverbindungen der Formel (IV)

R¹R²CH-CR³R⁴-MgZ (IV)

in der
- Z: ein Halogen aus der Gruppe Chlor, Brom oder Iod, bevorzugt Chlor oder Brom ist,
oder mit Verbindungen der Formel (IVa)

R¹R²CH-CR³R⁴-Li (IVa)

in der
- R¹, R², R³, R⁴: die oben unter Formel (I) angegebene Bedeutung haben,
umgesetzt werden.

Bevorzugt werden die Cyclopropanole aus Carbonsäureestern hergestellt. Ein bevorzugtes Verfahren ist somit dadurch gekennzeichnet, dass in den Verbindungen der Formel (I) X für OH und in den Verbindungen II Y für O steht.

Als bevorzugte Carbonsäureester R-COO-R⁵ kann eine Vielzahl von Verbindungen eingesetzt werden. Besonders bevorzugt werden Ester aliphatischer, cycloaliphatischer oder aromatischer Carbonsäuren eingesetzt. Solche Säuren sind beispielsweise Ameisensäure (R = H), Essigsäure (R = CH₃), Propionsäure (R = C₂H₅), Buttersäure und ihre verzweigten Isomeren (R = C₃H₇), Valeriansäure und ihre verzweigten Isomeren (R = C₄H₉), z. B. Pivalinsäure (R = tert-C₄H₉), Capronsäure (R = n-C₅H₁₁), Caprylsäure (R = C₇H₁₅), Caprinsäure (R = C₉H₁₉), Myristinsäure (R = C₁₃H₂₇), Palmitinsäure (R = C₁₅H₃₁), Stearinsäure (R = C₁₇H₃₅), wobei in allen Fällen auch verzweigte Isomere dieser Säuren Verwendung finden können, Cyclopropancarbonsäure, Cyclobutancarbonsäure, Cyclopentancarbonsäure, Cyclohexancarbonsäure usw. (R = entsprechender Cycloalkylrest), Benzoesäure und ihre substituierten Derivate (R = gegebenenfalls substituiertes C₆H₅), Naphthalin-1-und -2-carbonsäure (R = α-Naphthyl oder β-Naphthyl).

Die bevorzugt zu verwendenden Ester II entsprechend R⁵OH bzw. OR⁵ in den Verbindungen II für Y = O basieren auf Alkoholen wie Methanol, Ethanol, Propanol, Isopropanol, Butanol usw. entsprechend den Methyl-, Ethyl-, Propyl-, Isopropyl-, Butylestern. Die Bedeutung dieser Alkoholgruppe ist jedoch insofern nicht wesentlich, als die Gruppe OR⁵ als Abgangsgruppe fungiert und im synthetisierten Produkt nicht mehr vorhanden ist. Aus praktischen Erwägungen wird z.B. ein Ester auf Ethylalkoholbasis bevorzugt.

Gewöhnlich werden Niob- oder Tantalverbindungen der Formel (III) für die erfindungsgemäße Reaktion eingesetzt. Besonders bevorzugt ist die Verbindung III eine Tantalverbindung (M = Ta), da hiermit besonders gute Ausbeuten erzielt werden.

Die in einer bevorzugten Variante des Verfahrens einzusetzenden Niob- oder Tantalverbindungen sind bevorzugt Ethylate oder Isopropylate von Ta oder Nb. Somit wird ein Verfahren bevorzugt, das dadurch gekennzeichnet ist, dass in den Verbindungen III der Rest R⁷ eine Ethyl- oder eine Isopropylgruppe ist.

Besonders bevorzugt sind die in den neuen Verfahren einzusetzenden Verbindungen der Formel (III) Niobethoxid oder Niobisopropoxid oder Tantalethoxid oder Tantalisopropoxid, ganz besonders bevorzugt Tantalethoxid oder Tantalisopropoxid. Somit ist das bevorzugte Verfahren dadurch gekennzeichnet, dass in den Verbindungen der Formel (III) n = 0 ist.

Es können aber auch eine Reihe anderer Alkoxide eingesetzt werden; für Verbindungen der Formel (III), die auf Tantal (M = Ta) basieren, seien beispielhaft genannt Tantalmethoxid, Tantal-n-propoxid, Tantal-n-butoxid, Tantal-sec-butoxid, Tantal-tert-butoxid, Tantal-n-pentoxid, Tantal-n-hexoxid. Auch die Verwendung gemischter Halogenmetallalkoxide ist möglich. Wiederum am Beispiel der Tantalverbindungen seien beispielhaft genannt Chlortantaltetraethoxid, Chlortantaltetra-isopropoxid, Dichlortantaltriethoxid.

Als in den neuen Verfahren einzusetzende Grignard-Reagenzien IV eignet sich eine Vielzahl von Verbindungen. Als Voraussetzung gilt das Vorhandensein des Strukturelementes (V)

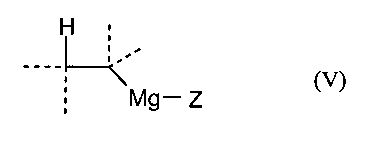

In den Grignard-Verbindungen der Formel (IV)

R¹R²CH-CR³R⁴-MgZ (IV)

ist Z ein Halogen aus der Gruppe Chlor, Brom oder Iod. Bevorzugt werden Grignard-Reagenzien mit Z = Br oder Cl eingesetzt, die bessere Umsätze erzielen. Im Einzelfall können statt der Grignard-Verbindungen auch die entsprechenden Lithiumorganyle eingesetzt werden (Li statt MgZ). Bevorzugt einzusetzende Grignard-Verbindungen sind dadurch gekennzeichnet, dass R¹, R³ und R⁴ = H sind. Beispielsweise sind zu nennen Ethylmagnesiumchlorid, n-Propylmagnesiumchlorid, n-Butylmagnesiumchlorid, n-Pentylmagnesiumchlorid, n-Hexylmagnesiumchlorid sowie die entsprechenden Bromide.

Es können auch insbesondere cyclische Grignardverbindungen wie Cyclopentylmagnesiumchlorid, Cyclohexylmagnesiumchlorid etc. eingesetzt werden.

Das neue Verfahren wird bevorzugt in zwei Schritten, beginnend bei einer Temperatur unter -20°C durchgeführt. Insbesondere wird die Verbindung der Formel (III) in einem geeigneten unten beschriebenen Lösungsmittel bei einer Temperatur von -78 bis -40°C, bevorzugt bei -78 bis -65°C vorgelegt und anschließend die Grignardverbindung IV in einem Lösungsmittel auf Etherbasis bei dieser Temperatur zudosiert. Nach einer Reaktionszeit bei dieser Temperatur von insbesondere bis zu 6 Stunden, bevorzugt ½ bis 3 Stunden, wird die Verbindung der Formel (II) (bevorzugt der Carbonsäureester, gegebenenfalls. mit Lösungsmittel verdünnt) zugetropft und das Reaktionsgemisch langsam, bevorzugt innerhalb von 5 Stunden, auf Raumtemperatur (ca. 23°C) erwärmt und die Reaktion zu Ende geführt und das Cyclopropanderivat isoliert.

Als Lösungsmittel auf Etherbasis werden bevorzugt niedere, aliphatische oder cyclische Ether eingesetzt, z.B. Diethylether, Di-n-propylether, Di-iso-propylether, Di-n-butylether, Tetrahydrofuran, Dioxan. Besonders bevorzugt wird Diethylether eingesetzt. Gegebenenfalls können andere, mit Diethylether mischbare Lösemittel in Mengen unter 50 Gew.-%, bezogen auf die GesamtLösungsmittelmenge, eingesetzt werden. Alle gegebenenfalls verwendeteten Lösungsmittel sind für die Reaktionsdurchführung wasserfrei, d. h. sorgfältig getrocknet (z.B. über Alkalimetalle oder Metallhydride destilliert) einzusetzen.

### Beispiele

Alle Versuche wurden unter Stickstoff durchgeführt. Tantalethoxid wurde frisch destilliert eingesetzt; Diethylether wurde über Natrium, Ethylacetat über Calciumchlorid destilliert.

### Beispiel 1

Aus 754 mg (31 mMol) Magnesium-Spänen und 3,38 g (31 mMol) Ethylbromid wurde in 15 ml Diethylether Ethylmagnesiumbromid hergestellt. Parallel wurden 4,064 g (10 mMol) Tantalethoxid in 80 ml Diethylether gelöst und auf -78°C gekühlt (Aceton/Trockeneis-Kühlmischung). Die Grignard-Lösung wurde zur Tantalethoxid-Lösung zugetropft und das Reaktionsgemisch 3 Stunden bei ca. -70°C gerührt. Danach wurden 881 mg (10 mmol) Ethylacetat zugetropft und durch Wegnahme des Kühlbades unter Rühren die Temperatur langsam auf ca. 23°C gesteigert. Insgesamt wurden 18 Stunden nachgerührt.

Zur Aufarbeitung wurden bei 0°C 25 ml 5%ige Salzsäure zugetropft. Die etherische Phase wurde abgetrennt und die wässrige Phase noch zweimal mit Diethylether ausgeschüttelt. Die vereinigten Etherphasen wurden über Na₂SO₄ getrocknet und eingedampft. Der Rückstand konnte säulenchromatographisch gereinigt werden; nach NMR-Analyse ergaben sich 20 % der theoretischen Ausbeute an 1-Methyl-cyclopropanol, bezogen auf Ethylacetat.

### Analysedaten:

¹H-NNM-Daten, δ (ppm gegen TMS; CDCl₃): 0,36 (m) 2H, 0,68 (m) 2H, 1,36 (s) CH₃.

### Beispiel 2

Aus 754 mg (31 mMol) Magnesium-Spänen und 3,38 g (31 mMol) Ethylbromid wurde in 15 ml Diethylether Ethylmagnesiumbromid hergestellt. Parallel wurden 3,182 g (10 mmol) Tantalethoxid in 80 ml Diethylether gelöst und auf-78°C gekühlt (Aceton/Trockeneis). Die Grignard-Lösung wurde zugetropft und das Reaktionsgemisch 1,5 Stunde bei ca. -70°C gerührt (Dunkelfärbung und brauner Niederschlag). Danach wurden 881 mg (10 mMol) Ethylacetat in 5 ml Diethylether zugetropft und durch Wegnahme des Kühlbades unter Rühren die Temperatur langsam auf ca. 23°C gesteigert. Insgesamt wurde ca. 18 Stunden nachgerührt.

Zur Aufarbeitung wurden bei 0°C 25 ml 5%ige Salzsäure zugetropft. Die etherische Phase wurde abgetrennt und die wässrige Phase noch zweimal mit Diethylether ausgeschüttelt. Die vereinigten Etherphasen wurden über Na₂SO₄ getrocknet und eingedampft. Der Rückstand konnte säulenchromatographisch gereinigt werden; nach NMR-Analyse ergaben sich 6 % der theoretischen Ausbeute an 1-Methyl-cyclopropanol, bezogen auf Ethylacetat.

### Analysedaten:

¹H-NMR-Daten des 1-Methyl-cyclopropanols, δ (ppm gegen TMS; CDCl₃): 0,36 (m) 2H, 0,68 (m) 2H, 1,36 (s) CH₃.

## Patentansprüche

1. Verfahren zur Herstellung von Cyclopropanderivaten der Formel (I), 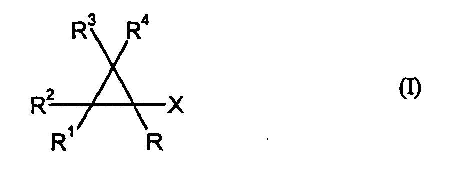 in der
R, R¹, R², R³ und R⁴ unabhängig voneinander Wasserstoff, C₁- bis C₁₈-Alkyl (einschließlich Cycloalkyl) oder C₆- bis C₁₀-Aryl bedeuten, wobei jeweils ein Rest aus R¹, R² und ein Rest aus R³, R⁴ gemeinsam einen Cycloalkan-Ring mit 4 bis 18 C-Atomen bilden können, X entweder OH oder YR⁵R⁶ bedeutet, wobei Y entweder O oder N bedeutet und für den Fall, dass Y = O ist, R⁶ entfällt und X zwangsläufig gleich OH ist, und wobei R⁵ und R⁶ unabhängig voneinander C₁- bis C₁₈-Alkyl oder C₆- bis C₁₀-Aryl bedeuten,
das **dadurch gekennzeichnet ist, dass** Alkancarbonsäurederivate der Formel (II) 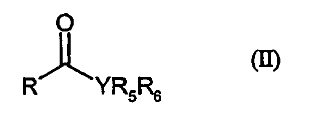 in der
Y, R, R⁵ und R⁶ die oben zu Formel (I) angegebene Bedeutung haben.
in Gegenwart von Verbindungen von Metallen der V. Nebengruppe (M = V, Nb, Ta) der Formel (III)
HalₙM(OR⁷)₅₋ₙ (III)
in der
Hal ein Halogen aus der Gruppe Chlor, Brom oder Iod, bevorzugt Chlor ist, n eine ganze Zahl von 0 bis 5, bevorzugt 0 oder 1, besonders bevorzugt 0 ist, M ein Element aus der Gruppe Vanadium, Niob und Tantal, bevorzugt Niob und Tantal, besonders bevorzugt Tantal ist und R⁷ ein C₁- bis C₈- Alkylrest ist
mit Grignardverbindungen der Formel (IV)
R¹R²CH-CR³R⁴-MgZ (IV)
in der
Z ein Halogen aus der Gruppe Chlor, Brom oder Iod, bevorzugt Chlor oder Brom ist,
oder mit Verbindungen der Formel (IVa)
R¹R²CH-CR³R⁴-Li (IVa)
in der
R¹, R², R³, R⁴ die oben unter Formel (I) angegebene Bedeutung haben,
umgesetzt werden.

2. Verfahren gemäß Anspruch 1, **dadurch gekennzeichnet, dass** in den Verbindungen der Formel (I) X = OH und Y = OR⁵ ist.

3. Verfahren gemäß den Ansprüchen 1 bis 2, **dadurch gekennzeichnet, dass** die Verbindung der Formel (III) eine Niob- oder Tantalverbindung ist (M = Nb oder Ta).

4. Verfahren gemäß wenigstens einem der Ansprüche 1 bis 3, **dadurch gekennzeichnet, dass** die Verbindung der Formel (III) eine Tantalverbindung ist (M = Ta).

5. Verfahren gemäß wenigstens einem der Ansprüche 1 bis 4, **dadurch gekennzeichnet, dass** in den Verbindungen der Formel (III) R⁷ für Ethyl oder Isopropyl steht.

6. Verfahren gemäß wenigstens einem der Ansprüche 1 bis 5, **dadurch gekennzeichnet, dass** in den Verbindungen der Formel (III) der Index n = 0 ist.

7. Verfahren gemäß wenigstens einem der Ansprüche 1 bis 5, **dadurch gekennzeichnet, dass** R¹, R³ und R⁴ für Wasserstoff stehen.

8. Verfahren gemäß wenigstens einem der Ansprüche 1 bis 5, **dadurch gekennzeichnet, dass** in der Verbindung der Formel (IV) Z für Br oder Cl steht.

9. Verwendung von Verbindungen der Formel (III)
HalₙM(OR⁷)₅₋ₙ (III)
zur Herstellung von Cyclopropanderivaten gemäß wenigstens einem der Ansprüche 1-8, wobei Hal, M, R⁷ und n die in Anspruch 1 angegebene Bedeutung haben.

## Claims

1. Process for preparing cyclopropane derivatives of the formula (I) 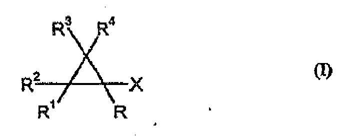 in which
R, R¹, R², R³ and R⁴ are each independently hydrogen, C₁- to C₁₈-alkyl (including cycloalkyl) or C₆- to C₁₀-aryl, where one radical from R¹, R² and one radical from R³, R⁴ together may in each case form a cycloalkane ring having 4 to 18 carbon atoms, X is either OH or YR⁵R⁶ where Y is either O or N and, in the case that Y = O, R⁶ is absent and X is necessarily OH, and where R⁵ and R⁶ are each independently C₁- to C₁₈-alkyl or C₆- to C₁₀-aryl,
which is **characterized in that** alkane carboxylic acid derivatives of the formula (II) 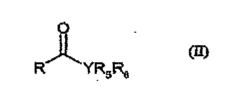 in which
Y, R, R⁵ and R⁶ are each as defined above for formula (I),
in the presence of compounds of metals of transition group V (M = V, Nb, Ta) of the formula (III)
HalₙM(OR⁷)₅₋ₙ (III)
in which
Hal is a halogen from the group of chlorine, bromine and iodine, preferably chlorine, n is an integer of 0 to 5, preferably 0 or 1, more preferably 0, M is an element from the group of vanadium, niobium and tantalum, preferably niobium and tantalum, more preferably tantalum, and R⁷ is a C₁- to C₈-alkyl radical,
are reacted with Grignard compounds of the formula (IV)
R¹R²CH-CR³R⁴-Mgz (IV)
in which
Z is a halogen from the group of chlorine, bromine and iodine, preferably chlorine and bromine,
or with compounds of the formula (IVa)
R¹R²CH-CR³R⁴-Li (IVa)
in which
R¹, R², R³, R⁴ are each as defined above under formula (I).

2. Process according to Claim 1, **characterized in that**, in the compounds of the formula (I), X = OH and Y = OR⁵.

3. Process according to Claims 1 and 2, **characterized in that** the compound of the formula (III) is a niobium or tantalum compound (M = Nb or Ta).

4. Process according to at least one of Claims 1 to 3, **characterized in that** the compound of the formula (III) is a tantalum compound (M = Ta).

5. Process according to at least one of Claims 1 to 4, **characterized in that** R⁷ in the compounds of the formula (III) is ethyl or isopropyl.

6. Process according to at least one of Claims 1 to 5, **characterized in that** the index n in the compounds of the formula (III) is 0.

7. Process according to at least one of Claims 1 to 5, **characterized in that** R¹, R³ and R⁴ are each hydrogen.

8. Process according to at least one of Claims 1 to 5, **characterized in that** Z in the compound of the formula (IV) is Br or Cl.

9. Use of compounds of the formula (III)
HalₙM(OR⁷)₅₋ₙ (III)
to prepare cyclopropane derivatives according to at least one of Claims 1 to 8, where Hal, M, R⁷ and n are each as defined in Claim 1.

## Revendications

1. Procédé pour la préparation de dérivés de cyclopropane de formule (I), 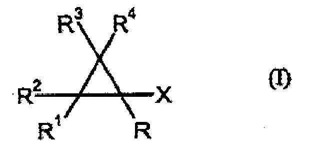 dans laquelle
R, R¹, R², R³ et R⁴ signifient, indépendamment les uns des autres, hydrogène, C₁ à C₁₈-alkyle (y compris cycloalkyle) ou C₆ à C₁₀-aryle, où à chaque fois un radical parmi R¹, R² et un radical parmi R³, R⁴ peuvent former ensemble un cycle cycloalcane comprenant 4 à 18 atomes de carbone, X signifie soit OH soit YR⁵R⁶, Y signifiant soit O soit N et, pour le cas où Y = O, R⁶ étant supprimé et X représentant obligatoirement OH, et R⁵ et R⁶ signifiant, indépendamment l'un de l'autre, C₁ à C₁₈-alkyle ou C₆ à C₁₀-aryle,
qui est **caractérisé en ce qu'**on transforme des dérivés d'acide alcanecarboxylique de formule (II) 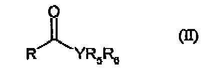 dans laquelle
Y, R, R⁵ et R⁶ ont la signification indiquée ci-dessus pour la formule (I),
en présence de composés de métaux du Vème groupe secondaire (M = V, Nb, Ta) de formule (III)
HalₙM(OR⁷)₅₋ₙ (III)
dans laquelle
Hal représente un halogène du groupe chlore, brome ou iode, de préférence chlore, n vaut un nombre entier de 0 à 5, de préférence 0. ou 1, de manière particulièrement préférée 0, M est un élément du groupe vanadium, niobium et tantale, de préférence niobium et tantale, de manière particulièrement préférée tantale et R⁷ représente un radical C₁ à C₈-alkyle
avec des composés de Grignard de formule (IV)
R¹R²CH-CR³R⁴-MgZ (IV)
dans laquelle
Z représente un halogène du groupe formé par chlore, brome ou iode, de préférence chlore ou brome,
ou avec des composés de formule (IVa)
R¹R²CH-CR³R⁴-Li (IVa)
dans laquelle
R¹, R², R³, R⁴ ont la signification indiquée ci-dessus pour la formule (I).

2. Procédé selon la revendication 1, **caractérisé en ce que** dans les composés de formule (I), X = OH et Y = OR⁵.

3. Procédé selon les revendications 1 à 2, **caractérisé en ce que** le composé de formule (III) est un composé du niobium ou du tantale (M = Nb ou Ta).

4. Procédé selon au moins l'une quelconque des revendications 1 à 3, **caractérisé en ce que** le composé de formule (III) est un composé du tantale (M = Ta).

5. Procédé selon au moins l'une quelconque des revendications 1 à 4, **caractérisé en ce que** dans les composés de formule (III), R⁷ représente éthyle ou isopropyle.

6. Procédé selon au moins l'une quelconque des revendications 1 à 5, **caractérisé en ce que** dans les composés de formule (III), l'indice n = 0.

7. Procédé selon au moins l'une quelconque des revendications 1 à 5, **caractérisé en ce que** R¹, R³ et R⁴ représentent hydrogène.

8. Procédé selon au moins l'une quelconque des revendications 1 à 5, **caractérisé en ce que** dans le composé de formule (IV), Z représente Br ou Cl.

9. Utilisation de composés de formule (III)
HalₙM(OR⁷)₅₋ₙ (III)
pour la préparation de dérivés de cyclopropane, selon au moins l'une quelconque des revendications 1 à 8, où Hal, M, R⁷ et n ont la signification indiquée dans la revendication 1.
